# EUROPEAN PATENT APPLICATION

(11) **EP 3 940 677 A1**
(43) Date of publication of application: **19.01.2022**
(21) Application number: 20186536.7
(22) Date of filing: 17.07.2020
(51) Int. Cl.: G09F 3/20, G09F 9/30, G09F 23/00, A61M 15/00

(54) **MEDICAMENT CONTAINER AND MEDICAMENT DELIVERY DEVICE**

(71) Applicant: Presspart GmbH & Co. KG, 34431 Marsberg (DE)
(72) Inventor: Seiler, Matthias, 40627 Düsseldorf (DE); Cott, Andreas, 40223 Düsseldorf (DE)
(74) Representative: Uexküll & Stolberg

(57) **Abstract**

A medicament container for storing a medicament, wherein the medicament container comprises a hollow can body (2) comprising an interior cavity (3) defined by a tubular sidewall (4), a bottom wall (5), and a top cover (8) closing opposite longitudinal ends of the sidewall (4) such that the sidewall (4) extends along a longitudinal axis (6) between the bottom wall (5) and the top cover (8). An electronic display element (13) is disposed on the can body (2) and is adapted to display in-formation content. An electronic unit (14) with a processing unit (16) is provided, wherein the processing unit is (16) in communication with the display element (13) for displaying said information content generated by the processing unit (16) .

## Description

### TECHNICAL FIELD

The invention relates to a medicament container for storing a medicament, wherein the medicament container comprises a hollow can body comprising an interior cavity defined by a tubular sidewall, a bottom wall and a top cover closing opposite longitudinal ends of the sidewall such that the sidewall extends along the longitudinal axis between the bottom wall and the top cover. The invention further relates to a medicament delivery device, in particular a metered dose inhaler.

### BACKGROUND OF THE INVENTION

The labeling of medicaments is an important issue for patients, doctors and suppliers. Conventionally, medicaments are distributed and provided to the patients in the form of a container in which the medicament is accommodated and stored. The medicament containers are often used in combination with a medicament delivery device, for example in the form of a metered dose inhaler. Metered dose inhalers (MDIs) are medicament delivery devices that are often used to deliver a pharmaceutical formulation including one or more pharmaceutically active compounds in the form of an aerosol to a human or other mammalian patient. Such MDIs comprise an actuation housing adapted to receive a medicament container in the form of canister. The canister is configured to move in the housing from a rest position to an activation position in which a valve of the container, positioned at a valve end of the container, is depressed against a bottom portion of the actuation housing such that an aerosol dose is released.

Each actuation of the MDI delivers one unit dose. The unit dose is expelled by the MDI and is taken into the body of the patient upon inhalation, via the nose or mouth. The pharmaceutical formulation is then delivered to the lungs of the patient. Metered dose inhalers are typically used for the treatment of respiratory infections and disorders including respiratory tract infections, obstructive lung disease, inflammatory lung disease and chronic obstructive pulmonary disease. Asthma treatment is a particularly common use of MDIs.

In most cases, it is important for patients to comply with the doctor's instructions for administering the medicament. Patients often receive a prescription from a medical practitioner and then purchase the medicament at a pharmacy. Administration instructions are often provided in the form of instruction leaflets. However, these leaflets often get lost and patients are hindered to comply with the treatment plan. On a regular basis, medicament related information is also printed on a sticker which is attached to the medicament container. However, such stickers are often not visible to the patient when the medicament container is received in the medicament delivery device, i.e., these important information are often not available to the patient when he administers the medicament.

Further, it can be vital for the patient to know about the quantity of the medicament in the medicament container, i.e., how many doses of the medicament are left in the medicament container, in particular when the medicament delivery device is administered on a daily basis. In order to address this problem, medicament delivery devices often include dose counters.

An MDI comprising a dose counter is for example disclosed in WO 2011/071788 A1. The inhaler comprises a mechanical dose counter having an indicator member which undergoes predetermined count-indicating motion when a dose is dispensed from the inhaler.

An alternative arrangement is disclosed in WO 2016/030844 A1. This document describes a metered dose inhaler having trigger members which upon depression of a canister trigger electrical switches thereby counting doses being released from the canister. Information regarding the remaining doses in the container are presented by the actuation housing which may include a processor and display means which, however, increase size and weight of the actuation housing.

It therefore shows that the presentation of container related information to the patient that is using the medicament delivery device is often connected with increased dimensions of the medicament delivery device and high production costs. At the same time, the medication delivery device is often inconvenient to hold due to the increase in size.

### SUMMARY OF THE INVENTION

It is an object of the present invention to provide a solution for presenting container related information in an efficient manner, in particular with respect to assembly space, handling and costs.

The above-mentioned object is achieved by a medicament container comprising the features of claim 1 and by a medicament delivery device as defined in claims 12. Preferred embodiments of the invention are set out in the dependent claims. According to an embodiment of the present invention, a display element is disposed on the can body, wherein the display element is adapted to display information content. The medicament container further comprises an electronic unit with a processing unit which is in data communication with the display element and generates signals which cause the display element to display the information content.

The display element is an electronic display, in particular a digital display, and may be provided in the form of a display panel. LED (light emitting diode), OLED (organic light emitting diode), LCD (liquid crystal display) or electronic paper based displays are merely mentioned by way of example for suitable displays, only.

The information content presented on the display element may be presented in any suitable form. For example, information may be displayed in the form of numbers, images, including for example, still or moving color images. Data or information presented to the user may in particular include user and/or container related information, such as content of the medicament container, the amount of medicament remaining in the container, use instructions for the medicament, patient data, delivery information and locations (e.g. for intra-hospital logistics or e-commerce pharmacies), matching codes for e-recipes or live advises for handling the container. The displayed information may also relate to other products made by the same manufacturer, or even unrelated subject matter.

The electronic unit to operate the display element may be provided separately from the display element. The electronic unit comprises the necessary electronic components to operate the display element, e.g. a processor, the required circuitry, a power source (battery) and memory components.

The electronic unit may, for example, be provided in the form of a pre-assembled unit with the processing unit and respective circuitry and power supply in the form of a battery unit to operate the display element, wherein the electronic unit is electronically connected to the display element, e.g. by wiring.

Alternatively, the electronic unit may be integral with the display element, i.e. the display element may be a pre-assembled unit comprising the electronic unit. The pre-assembled unit may be mounted on the battery can by suitable means, e.g. by bonding.

Providing information directly on the medicament container allows reducing the overall dimensions of the medicament delivery device. Further, providing a medicament container with an electronic display element effectively uses the current trend toward miniaturization of components in a cost effective manner. Since the display element is an electronic display, the presentation of information is flexible, i.e., the display element allows for higher information content, in particular when compared to regular dose counters, stickers, use instructions leaflets, etc..

According to a further embodiment of the present invention, the display element is provided as a flexible display element. A flexible display is an electronic visual display which is flexible in nature; as opposed to the more prevalent traditional flat screen displays used in most electronics devices. The flexible display may be provided, e.g. in the form of a flexible OLED-based display (organic light emitting diode display) or in the form of flexible electronic paper based display. Flexible displays have the advantage that they may be attached to the outer surface of the tubular sidewall thereby conforming to the tubular, i.e., rounded shape of the sidewall.

According to a further embodiment of the present invention, the display element is configured as a touch screen display for the input and output of information and/or instructions. A touch screen display may, for example, enable a doctor to input additional instructions for the patient. Further, a touch screen display allows the user of the medicament container to interact with the container. In contrast to stickers with instructions printed thereon, a larger quantity of information may be presented to the user, e.g. by an interactive program running on the processor, which provides use instructions for the medicament in an interactive manner.

According to further embodiment of the present invention the electronic unit further comprises a memory unit for storing and/or outputting data in/from the electronic unit. The electronic unit may receive instructions or feedback input by the user with the touch screen display or alternatively by an additional data port, such as a micro-USB port provided by the electronic unit. Notably, the memory unit may be used to store use information regarding the use of the medicament container. Thereby, administration of the medicament can effectively be monitored and the patient can be supported in following his treatment plan. These data can, of course, be retrieved from the memory unit and further processed, e.g. by the medical practitioner at a later stage for optimizing the treatment plan of the patient. The data can also be read by displaying them on the display element.

According to further embodiment of the present invention, the display element is disposed on the outside of the tubular side wall. The display element may be attached to the can body in such a manner that it extends along the outer circumferential surface of the can body, either completely along the outer circumferential surface or less. The display element can extend on the outer surface of the side wall between the bottom wall and the top cover. This extension may reach from the bottom wall to the top cover or less.

A further embodiment of the present invention provides that the display element is provided in a recessed portion of the outer surface of the tubular side wall. Preferably, the outer surface of the display element lies flush with the outer surface the side wall. Alternatively, the display element may also be provided on a cut-out portion of the side wall, i.e. the side wall surrounds the display element in a frame-like manner.

Generally, the display element may be mounted on the can body by suitable attachment means. For example, the display element may be attached to the can body by bonding. Suitable bonding methods are, for example, Optical Clear Adhesive (OCA) Bonding or Liquid Optically Clear Adhesive (LOCA) Bonding.

According to a further embodiment of the present invention, the electronic unit is at least partly located at the bottom section of the can body, i.e. in a region of the bottom wall. As indicated previously, the electronic unit may be provided as a pre-assembled unit attached to the can body. The pre-assembled unit may be located underneath a dome-shaped portion of the bottom wall, i.e., the electronic unit is located on the side of the bottom wall which is facing away from the interior cavity. The electronic unit is electronically coupled to the display element by wiring or other suitable means. The electronic unit may also be provided in the form of a cap fastened to the can body.

According to further embodiment of the present invention, the medicament container comprises a dispensing unit configured to dispense the medicament from the medicament container upon actuation of the dispensing unit. Further, sensing means adapted to sense one or more conditions of the dispensing unit and/or the medicament container are provided. The sensing means are electronically coupled to the electronic unit to further process the data from the sensing means. For example, in one embodiment of the present invention, the dispensing unit is provided in the form of a valve assembly, i.e. the top cover comprises a valve whose condition (e.g. opening period and/or opening degree) is sensed by the sensing means. The sensing means may include for example pressure sensors, actuation sensors, flow sensors or the like. The processor may determine on the basis of the sensing means the amount of medicament dispensed from the container by further processing the data received from the sensing means. The remaining doses in the medicament container may be determined by the processing unit and this information may be presented to the user on the display element. In comparison to, for example, traditional MDIs with dose counters on the actuation housing, the presentation of information relating to the amount of medicament left in the medicament container is transferred to the medicament container. Dose counting mechanisms and respective display elements on the actuation housing may be dispensed.

According to a further embodiment of the present invention, the medicament container is formed as a metered dose inhaler (MDI) container. As indicated previously, these MDI containers include a top cover with a valve. The top cover is usually attached to the can body by a crimped ferrule and is configured to be inserted into a metered dose inhaler. The valve is opened upon depressing the valve toward the interior of the container.

According to a further embodiment of the present invention, the can body is a deep drawn part forming the tubular side wall in the bottom wall of the medicament container, i.e. the side wall and the bottom wall of the medicament container are formed in one piece. Preferably, the can body is basically of cylindrical shape and has a dome-shaped bottom wall which is inwardly tipped. The term cylindrical is not meant to be restricted to a circular cylinder, although this form might be a preferred one. The term cylindrical may also include cylinders having a polygonal or elliptical base.

Deep-drawn medicament containers are typically deep drawn from metal cups, which are formed from metal sheets, often also referred-to as metal blanks. As defined in industry norm DIN 8584, the deep drawing process is a forming process which occurs under a combination of tensile and compressive conditions. A flat sheet metal blank is formed into a hollow body open on at least one side or a hollow body is formed into a hollow body with a smaller cross-section. A typical deep drawing process is e.g. described in US Patent 2 989 019, which is herewith incorporated by reference.

According to a further embodiment of the present invention, the medicament container contains a pharmaceutical product.

The present invention also relates to a medicament delivery device comprising a medicament container as described herein. Medicament delivery devices are in particular characterized by a housing arranged to accommodate a medicament container and by actuation means that when actuated by the user interact with the medicament container, e.g. a dispensing unit of the medicament container, such that the content of the medicament container is dispensed.

According to a further embodiment of the present invention, the medicament delivery device is a metered dose inhaler for dispensing aerosol doses comprising an actuation housing and a medicament container as described herein. The medicament container is received in the actuation housing, e.g. in a receptacle of the actuation housing, and is configured to move in the housing from a rest position to an activation position in which the medicament container is configured to dispense medication from the interior cavity. The medicament container is configured to move from a rest position to an activation position in which a valve of the medicament container, e.g. positioned at a valve end of the medicament container, is depressed against a bottom portion of the actuation housing such that the valve is opened and an aerosol dose is released. The valve end may be provided at the top cover in which a valve is located.

According to a further embodiment of the invention, the actuation housing comprises a window, a cut-out, an opening or the like which is aligned with the display element of the medicament container body such that the information content displayed on the display element is visible through the window.

According to a further embodiment of the invention, the actuation housing further comprises a mouth piece for inhalation of the aerosol.

### BRIEF DESCRIPTION OF THE FIGURES

The invention will now be described referring to exemplary embodiments shown in the Figures in which:
- Fig. 1: shows a sectional view of a medicament container in accordance with a first embodiment of the invention;
- Fig. 2: the medicament container of Fig. 1 in a perspective view; and
- Fig. 3: a metered dose inhaler in accordance with a further embodiment of the invention.

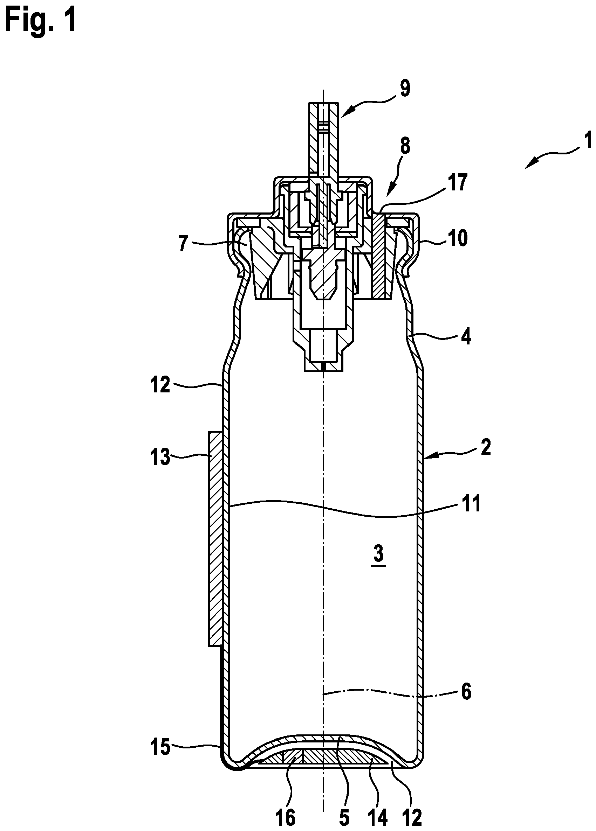

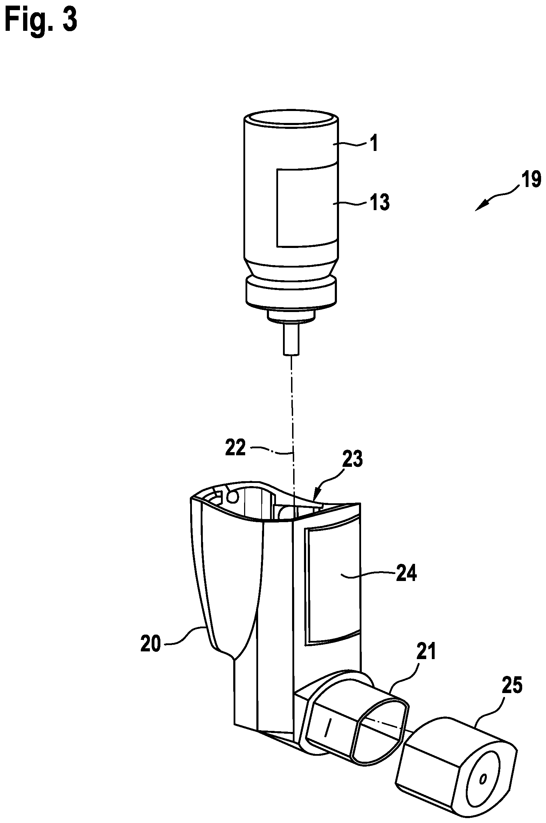

**Fig. 1** shows medicament container 1 in the form of an aerosol canister (MDI container) for use with an actuation housing (Figure 3) to form a metered dose inhaler as shown in Fig. 3. This medicament container is filled with a medicament in the form of an aerosol.

The medicament container 1 comprises a can body 2 with an interior cavity 3. The interior cavity is defined by a tubular side wall 4 of the can body 2 and a bottom wall 5 that is integrally formed with the tubular side wall 4.

The can body 2 is a hollow deep-drawn article with a longitudinal axis 6 which extends from the bottom wall 5 towards an open end 7 of the can body 2. The container 1 has a hollow cylindrical shape, i.e., when viewed along the longitudinal axial 6, the container has a circular cross section. The open end 7 is closed with a top cover 8 which comprises a valve 9. When the valve 9 is depressed, i.e., displaced toward the interior cavity 3, the valve 9 is opened so that the aerosol, which is stored within the container 1 under pressure, is dispensed through the valve 9.

The top cover 8 is attached to the can body 2 by crimping, i.e. a ferrule 10 is crimped over the open end 7 of the can body 2 and thereby fixes the top cover 8 to the can body 2.

The can body 2 has an inner surface 11 and an outer surface 12. The bottom wall 5 has a dome shape and protrudes toward the interior cavity 3.

A display element 13 in the form of a flexible OLED display is attached to the outer surface 12 of the can body 2 by bonding. This electronic display element 13 is connected to an electronic unit 14 located underneath the dome-shaped bottom wall 5 by wiring 15. The electronic unit 14 is attached to the outer surface 12 of the bottom wall 5, i.e., on the side of the bottom wall facing away from the interior cavity 3.

The display element 13 is adapted to display information content in the form of letters, still or moving images, numbers or the like. In order to operate and control the display element 13, the electronic unit 14 comprises a processing unit 16. The processing unit 16 generates signals which cause the display element 13 to display the information content.

The electronic unit 14 further comprises a memory unit (not shown) which can store and output data as well as a power source in the form of a battery (not shown).

The top cover 8 further comprises sensing means 17. In the illustrated embodiment, the sensing means 17 is an actuation sensor to determine when the valve 9 is actuated. The actuation sensor 17 is electronically connected (not shown) to the electronic unit 14.

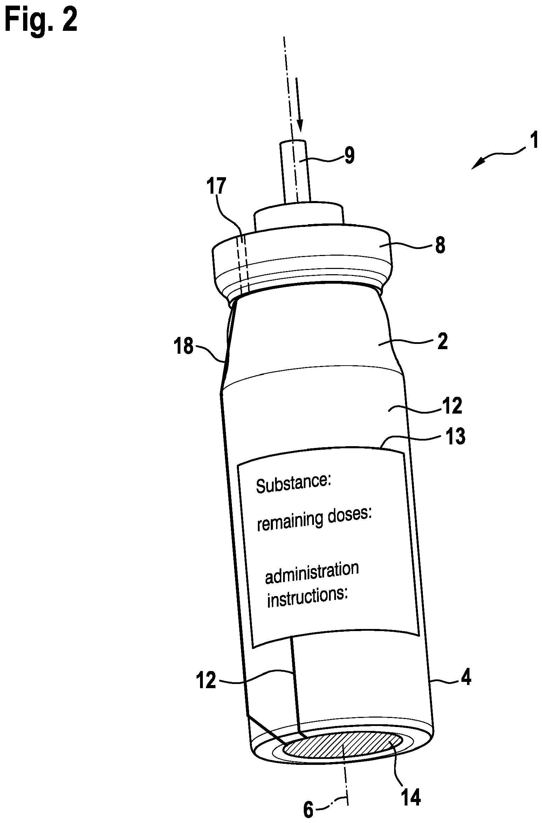

**Fig. 2** is a perspective view of the medicament container 1 of Figure 1. The display element 13 is disposed on the outer surface of the tubular side wall 4 such as to extend in circumferential direction around the longitudinal axis 6, i.e., along the outer circumferential surface of the tubular side wall 4. The display element 13 is connected to the electronic unit 14 located in the bottom section of the medicament container by wiring 12. As indicated previously, the display element 13 is operated by the electronic unit 14.

The electronic unit 14 stores data relating to the medicament container such as substance stored within the medicament container and initial quantity. The actuation sensor 17 (indicated by broken lines) in the top cover 8 is electronically connected to the electronic unit 14, in particular its processing unit, by wiring 18. When the vale 9 is depressed toward the interior of the can body, the valve 9 is opened. The actuation sensor 17 senses the actuation of the valve 9 and transmits its sensor data to the processing unit of the electronic unit 14. The electronic unit 14 determines on the basis of the data provided by the actuation sensor the opening period and the opening degree of the valve 9. On that basis the electronic unit 14 determines the quantity of the aerosol dispensed from the medicament container and calculates the remaining doses in the medicament container. This information, among others such as administration instructions and substance, is displayed on the display element 13 and is updated upon use of the metered dose inhaler.

The display element 13 is configured as a touch display so that the display element can output information visually. The touch display 13 can also be used for inputting information into the electronic unit 14. For example, a doctor can use the display element 13 to input administration instructions for later use of the medicament container by the patient. These instructions are stored in the memory unit of the electronic unit. When the patient receives the medicament container, the administration instructions previously input by the doctor may be displayed to the patient by the display element 13.

**Fig. 3** is a perspective view of components of a metered dose inhaler (MDI) 19 for dispensing aerosol doses. The metered dose inhaler 19 comprises an actuation housing 20 with a mouthpiece 21 through which a patient inhales a pharmaceutical formulation contained in the medicament container 1. The actuation housing 20 has a longitudinal axis 22 along which the actuation housing 20 is configured to receive the medicament container 1 with the valve end first in a receptacle 23.

The actuation housing 20 has a window 24 or opening in the sidewall of the actuation housing 20 forming the receptacle 23. When the medicament container 1 is received in the receptacle 23, the display element 13 is aligned with the window 24 so that the display element 13 can be viewed through the window 24.

To ensure that the medicament container 1 is properly aligned with the window 24, guiding and alignment means (not shown), may be provided on the outer surface of the medicament container and on the inside of the receptacle 23.

During non-use of the MDI 19, the mouthpiece 21 may be covered by a cap 25.

### Reference numerals

- 1: medicament container (aerosol canister)
- 2: can body
- 3: interior cavity
- 4: tubular sidewall
- 5: bottom wall
- 6: longitudinal axis
- 7: open end
- 8: top cover
- 9: valve
- 10: ferrule
- 11: inner surface
- 12: outer surface
- 13: display element
- 14: electronic unit
- 15: wiring
- 16: processing unit
- 17: sensing means (actuation sensor)
- 18: wiring
- 19: metered dose inhaler (MDI)
- 20: actuation housing
- 21: mouthpiece
- 22: longitudinal axis
- 23: receptacle
- 24: window
- 25: cap

## Claims

1. A medicament container for storing a medicament, wherein the medicament container comprises a hollow can body (2) comprising an interior cavity (3) defined by
a tubular sidewall (4),
a bottom wall (5), and
a top cover (8) closing opposite longitudinal ends of the sidewall (4) such that the sidewall (4) extends along a longitudinal axis (6) between the bottom wall (5) and the top cover (8),
**characterized by** an electronic display element (13) disposed on the can body (2), wherein the display element (13) is adapted to display information content; and by an electronic unit (14) with a processing unit (16), wherein the processing unit is (16) in communication with the display element (13) for displaying said information content generated by the processing unit (16).

2. Medicament container according to claim 1, **characterized in that** the display element is a flexible display element (13).

3. Medicament container according to claims 1 or 2, **characterized in that** the display element (13) is configured as a touch screen display for the input and output of information and/or instructions.

4. Medicament container according to any of the preceding claims, **characterized in that** the electronic unit (14) further comprises a memory unit for storing and/or outputting data.

5. Medicament container according to any of the preceding claims, **characterized in that** the display element (13) is disposed on the tubular sidewall (4), in particular in a recessed portion of the outer surface (12) of the tubular sidewall (4).

6. Medicament container according to any of the preceding claims, **characterized in that** the electronic unit (14) is at least partly located at a bottom section of the can body (2).

7. Medicament container according to any of the preceding claims, **characterized in that** the electronic unit (14) is provided underneath a dome-shaped portion of the bottom wall (5) on the side of the bottom wall (5) facing away from the interior cavity (3).

8. Medicament container according to any of the preceding claims, **characterized by** a dispensing unit (9) configured to dispense the content of the medicament container (1) and by sensing means (17) adapted to sense on or more conditions of the medicament container, in particular of the dispensing unit (9).

9. Medicament container according to any of the preceding claims, **characterized in that** the medicament container is configured as a metered dose inhaler (MDI) container.

10. Medicament container according to any of the preceding claims, wherein the medicament container includes a deep-drawn part forming the tubular sidewall (4) and the bottom wall (5).

11. Medicament container according to any of the preceding claims, wherein the medicament container contains a pharmaceutical product, preferably in the form of an aerosol.

12. Medicament delivery device comprising a medicament container according to any of the preceding claims.

13. Medicament delivery device according to claim 12, wherein the medicament delivery device is a metered dose inhaler (MDI)(19) for dispensing aerosol doses comprising an actuation housing (20) in which the medicament container (1) is received, wherein the medicament container (1) contains an aerosol and is configured to move from a rest position to an activation position in which a valve (9) of the medicament container is depressed against a bottom portion of the actuation housing (2) such that an aerosol dose is released.

14. Medicament delivery device according to claims 12 or 13, **characterized in that** the actuation housing (20) comprises a window (24) which is aligned with the display element (13) of the medicament container (1) such that the information content displayed on the display element (13) is visible through the window (24).

15. Medicament delivery device according to any of claims 13 or 14 when depending on claim 13, **characterized in that** the actuation housing (2) further comprises a mouthpiece (21) for inhalation of the aerosol
